# FASCICULE DE BREVET EUROPEEN

(11) **EP 2 997 078 B1**
(45) Date de publication et mention de la délivrance du brevet: **26.06.2019**
(21) Numéro de dépôt: 14725131.8
(22) Date de dépôt: 16.05.2014
(51) Int. Cl.: C08J 3/24, A61K 8/88, C08G 69/14, C08G 73/14

(54) **PROCÉDÉ DE RÉTICULATION EN SURFACE DE PARTICULES DE POLYMÈRE**
VERFAHREN ZUR OBERFLÄCHENVERNETZUNG VON POLYMERTEILCHEN
PROCESS OF SURFACE CROSS-LINKING OF POLYMER PARTICLES

(30) Priorité: 17.05.2013 FR 1354473
(43) Date de publication de la demande: 23.03.2016
(73) Titulaire: Arkema France, 92700 Colombes (FR)
(72) Inventeur: CAMMAGE, Geoffroy, F-76000 Rouen (FR); DUFAURE, Nicolas, F-27300 Bernay (FR); LE, Guillaume, F-14460 Colombelles (FR); MATHIEU, Cyrille, 69600 Oullins (FR)
(74) Mandataire: Casalonga
(86) Numéro de dépôt international: PCT/EP2014/060105
(87) Numéro de publication internationale: WO 2014/184351

(56) Documents cités:
- EP-A1- 1 661 546
- DE-A1- 10 251 790
- FR-A1- 2 588 262
- FR-A1- 2 910 907
- US-A1- 2008 182 960

## Description

L'invention porte sur un procédé de réticulation en surface de particules de polymères. L'invention porte également sur des particules de polymère partiellement réticulé obtenu par ledit procédé ainsi que sur l'utilisation de ces particules dans le domaine des composites, des adhésifs structuraux, des revêtements et de la cosmétique. La présente invention concerne enfin une composition comprenant lesdites particules.

Il est connu depuis de nombreuses années d'incorporer à des matériaux composites des poudres de polymère, en général des polyamides, agissant comme renfort choc placées entre les couches du matériau composite, ou encore agissant comme charge inerte. Ces poudres de polymère permettent d'améliorer les propriétés mécaniques des matériaux, elles peuvent notamment jouer le rôle de durcisseur ou d'agents anti-délamination.

Il est aussi connu d'utiliser ces poudres de polymère dans des compositions cosmétiques, et notamment dans des produits de soin, de maquillage, des produits parfumants ou encore d'hygiène corporelle. Ces poudres confèrent de nombreuses propriétés aux compositions cosmétiques les contenant, et notamment un toucher doux, un fini poudré, une diminution de la sensation de gras parfois associée aux crèmes, ou encore un effet matifiant.

De plus, ces poudres de polymères peuvent présenter une structure poreuse, elles permettent ainsi une vectorisation des principes actifs et une libération contrôlée.

Ainsi, le document FR 2 910 907 concerne notamment un procédé de préparation par polymérisation anionique d'une nouvelle poudre de polyamide 12, polyamide 6 ou de polyamide 6/12 ensemencée par une charge inorganique. Par ailleurs, le document DE 102 51 790 porte sur une composition comprenant entre 88 et 99,99% en poids d'un polymère choisi parmi un polyamide, un composé d'un polyamide et un mélange de ceux-ci, et entre 0,01 et 0,25% en poids d'un auxiliaire d'écoulement, comme par exemple la silice. Quant au document EP 1 661 546, il divulgue des compositions cosmétiques pour soin et/ou maquillage qui sont des émulsions comprenant principalement une phase aqueuse et une phase grasse et comprenant en plus une poudre fine et poreuse.

Ces applications demandent des combinaisons de propriétés spécifiques, telles qu'une faible reprise en humidité, une bonne ductilité, associées à une température de fusion élevée. Les poudres de polymères de l'art antérieur présentent un compromis intéressant en reprise en humidité et de ductilité, mais elles présentent l'inconvénient d'avoir une température de fusion (Tf) relativement basse, souvent inférieure à 180°C.

En effet, l'utilisation de ces poudres est limitée, lorsqu'un procédé de formulation nécessite une étape à haute température.

Des phénomènes de coalescence peuvent être observés, conduisant à une déformation des particules, en termes de forme et de taille, détériorant de manière irréversible les effets mécaniques attendus.

Ainsi, il existe un réel besoin de particules de polymère présentant une tenue thermique améliorée, une faible reprise en humidité, une bonne résistance chimique, notamment vis à vis des solvants, et dont les propriétés physico-chimiques sont conservées au cours de la préparation ou de la formulation du matériau.

L'invention porte sur un procédé de réticulation en surface d'un polymère, sous la forme de particule, possédant une ou plusieurs fonctions à hydrogène labile comprenant une étape mettant en oeuvre un agent de réticulation comprenant au moins 2 fonctions susceptibles de réagir avec les fonctions à hydrogène labile du polymère, le procédé de réticulation se faisant à une température inférieure au point de fusion du polymère.

L'invention porte également sur un procédé de polymérisation incorporant le procédé de réticulation pré-cité.

L'invention vise aussi des particules de poudre de polymère partiellement réticulé.

La présente invention concerne enfin une composition comprenant ladite poudre.

L'invention vise enfin l'utilisation de ces poudres dans le domaine de la cosmétique et dans le domaine des composites.

D'autres caractéristiques, aspects, objets et avantages de la présente invention apparaîtront encore plus clairement à la lecture de la description et des exemples qui suivent.

D'autre part, tout intervalle de valeurs désigné par l'expression "entre a et b" représente le domaine de valeurs allant de plus de a à moins de b (c'est-à-dire bornes a et b exclues), tandis que tout intervalle de valeurs désigné par l'expression "de a à b" signifie le domaine de valeurs allant de a jusqu'à b (c'est-à-dire incluant les bornes strictes a et b).

Au sens de la présente invention, les termes « particule » et « poudre » sont utilisés indifféremment l'un de l'autre pour désigner la forme du polymère. Le terme particule sera utilisé pour désigner la forme du polymère de manière individualisée, et le terme poudre sera utilisé pour désigner un ensemble de particules de polymère.

Les particules de polymère selon l'invention se présentent à l'état solide.

### Procédé de réticulation

L'invention porte sur un procédé de réticulation en surface d'un polymère, sous forme de particule possédant une ou plusieurs fonctions à hydrogène labile, ledit polymère étant choisi parmi les polymères comportant une ou plusieurs fonctions amide, les polyuréthanes, les polyamines et un mélange de ces polymères, comprenant une étape mettant en oeuvre un agent de réticulation comprenant au moins 2 fonctions susceptibles de réagir avec les fonctions à hydrogène labile du polymère, le procédé de réticulation se faisant à une température inférieure au point de fusion du polymère.

### Particules

Les matières premières du procédé de réticulation selon l'invention sont des polymères possédant une ou plusieurs fonctions à hydrogène labile.

Par « fonction à hydrogène labile », on entend selon l'invention une fonction capable, après départ d'un atome d'hydrogène, de former des liaisons covalentes.

Ainsi, après une éventuelle réaction acido-basique provoquée par exemple par un catalyseur, la fonction à hydrogène labile devient nucléophile et réagit avec une fonction électrophile de l'agent réticulant, pour conduire à la formation d'une liaison covalente.

On peut citer à titre d'exemple de telles fonctions les groupes hydroxyle(-OH), amine primaire (-NH₂) ou amine secondaire (-NHR), amide primaire (-CONH-), urée (-NHCONH-), uréthane (-NHCOO-), ou encore les groupes thiol (-SH).

Les polymères ayant une ou plusieurs fonction(s) à hydrogène labile selon l'invention choisis parmi les polymères comportant une ou plusieurs fonctions amides, en particulier les polyamides et les polyamides-imides, les polyuréthanes, les polyamines et un mélange de ces polymères.

De manière particulièrement préférée, le polymère ayant une ou plusieurs fonction(s) à hydrogène labile selon l'invention sont choisis parmi les polyamides et les polyamides-imides et leur mélange, les fonctions à hydrogène labile sont alors des fonctions amides. Les polyamides sont particulièrement préférés.

Le ou les polyamide(s) particulièrement préféré(s) selon l'invention peuvent être choisis parmi des polyamides obtenus par polycondensation ou par polymérisation anionique de lactames, d'aminoacides ou de diacides et de diamines.

Par polyamide, on entend au sens de la présente invention un homopolyamide, c'est-à-dire un polyamide obtenu à partir d'un seul type de monomère ou un copolyamide, c'est-à-dire un polyamide obtenu à partir de plusieurs types de monomères différents.

Le motif répétitif constituant le polyamide peut être choisi parmi des motifs dérivés d'un aminoacide, un lactame et un motif répondant à la formule (diamine en Ca).(diacide en Cb), avec a représentant le nombre d'atomes de carbone de la diamine et b représentant le nombre d'atomes de carbone du diacide, a et b étant chacun allant de 4 à 36.

Lorsque le motif représente un motif dérivé d'un aminoacide, il peut être choisi parmi l'acide 9-aminononanoïque (A=9), l'acide 10-aminodécanoïque (A=10), l'acide 12-aminododécanoïque (A=12) et l'acide 11-aminoundécanoïque (A=11) ainsi que ses dérivés, notamment l'acide N-heptyl-11-aminoundécanoïque.

Lorsque le motif représente un motif dérivé d'un un lactame, il peut être choisi parmi la pyrrolidinone, la 2-pipéridinone, le caprolactame (A=6), l'énantholactame, le caprylolactame, le pelargolactame, le décanolactame, l'undecanolactame, et le lauryllactame (A=12).

Lorsque le motif représente un motif dérivé d'un motif répondant à la formule (diamine en Ca).(diacide en Cb), le motif (diamine en Ca) est choisi parmi les diamines aliphatiques, linéaires ou ramifiées, les diamines cycloaliphatiques et les diamines alkylaromatiques.

Lorsque la diamine est aliphatique et linéaire, de formule H₂N-(CH₂)ₐ-NH₂, le monomère (diamine en Ca) est préférentiellement choisi parmi la butanediamine (a=4), la pentanediamine (a=5), la hexanediamine (a=6), l'heptanediamine (a=7), l'octanediamine (a=8), la nonanediamine (a=9), la décanediamine (a=10), l'undécanediamine (a=11), la dodécanediamine (a=12), la tridécanediamine (a=13), la tétradécanediamine (a=14), l'hexadécanediamine (a=16), l'octadécanediamine (a=18), l'octadécènediamine (a=18), l'eicosanediamine (a=20), la docosanediamine (a=22) et les diamines obtenues à partir d'acides gras.

Lorsque la diamine est aliphatique et ramifiée, elle peut comporter un ou plusieurs substituants méthyle ou éthyle sur la chaîne principale. Par exemple, le monomère (diamine en Ca) peut avantageusement être choisi parmi la 2,2,4-triméthyl-1,6-hexanediamine, la 2,4,4-triméthyl-1,6-hexanediamine, le 1,3-diaminopentane, la 2-méthyl-1,5-pentanediamine, la 2-méthyl-1,8-octanediamine.

Lorsque le monomère (diamine en Ca) est cycloaliphatique, il est préférentiellement choisi parmi la bis(3,5-dialkyl-4-aminocyclohexyl) méthane, la bis(3,5-dialkyl-4-aminocyclohexyl) éthane, la bis(3,5-dialkyl-4-aminocyclo-hexyl)propane, la bis(3,5-dialkyl-4-aminocyclo-hexyl)butane, la bis-(3-méthyl-4-amino cyclohexyl)-méthane (BMACM ou MACM), la p-bis(aminocyclohexyl) -méthane (PACM) et l'isopropylidènedi(cyclohexylamine) (PACP). Il peut également comporter les squelettes carbonés suivants : norbornyl méthane, cyclohexylméthane, dicyclohexylpropane, di(méthyl cyclohexyl), di(méthylcyclohexyl) propane. Une liste non-exhaustive de ces diamines cycloaliphatiques est donnée dans la publication "Cycloaliphatic Aminés" (Encyclopaedia of Chemical Technology, Kirk-Othmer, 4th Edition (1992), pp. 386-405).

Lorsque le monomère (diamine en Ca) est alkylaromatique, il est préférentiellement choisi parmi la 1,3-xylylène diamine et la 1,4-xylylène diamine.

Lorsque le motif est un motif répondant à la formule (diamine en Ca).(diacide en Cb), le motif (diacide en Cb) est choisi parmi les diacides aliphatiques, linéaires ou ramifiés, les diacides cycloaliphatiques et les diacides aromatiques.

Lorsque le monomère (diacide en Cb) est aliphatique et linéaire, il est choisi parmi l'acide succinique (b=4), l'acide pentanedioïque (b=5), l'acide adipique (b=6), l'acide heptanedioïque (b=7), l'acide octanedioïque (b=8), l'acide azélaïque (b=9), l'acide sébacique (b=10), l'acide undécanedioïque (b=11), l'acide dodécanedioïque (b=12), l'acide brassylique (b=13), l'acide tetradécanedioïque (b=14), l'acide hexadécanedioïque (b=16), l'acide octadécanedioïque (b=18), l'acide octadécènedioïque (b=18), l'acide eicosanedioïque (b=20), l'acide docosanedioïque (b=22) et les dimères d'acides gras contenant 36 carbones.

Les dimères d'acides gras mentionnés ci-dessus sont des acides gras dimérisés obtenus par oligomérisation ou polymérisation d'acides gras monobasiques insaturés à longue chaîne hydrocarbonée (tels que l'acide linoléïque et l'acide oléïque), comme décrit notamment dans le document EP 0 471 566.

Lorsque le diacide est cycloaliphatique, il peut comporter les squelettes carbonés suivants : norbornyl méthane, cyclohexylméthane, dicyclohexylméthane, dicyclohexylpropane, di(méthylcyclohexyl), di(methylcyclohexyl)propane.

Lorsque le diacide est aromatique, il est préférentiellement choisi parmi l'acide téréphtalique (noté T), isophtalique (noté I) et les diacides naphtaléniques.

Les polyamides peuvent être cristallins ou amorphes et transparents.

De préférence, les polyamides selon l'invention sont choisis parmi les polyamides aliphatiques, plus particulièrement ceux dont la longueur de chaîne des motifs va de 4 à 18, plus particulièrement de 4 à 12. Plus préférentiellement, les polyamides selon l'invention sont choisis parmi les PA6, PA11, PA12, PA6/12, PA6.12, PA6.6, PA8, PA4, PA 4.6, PA 10.10, PA 6.10, PA 10.12 ; et un mélange de ces polymères. De préférence, le polyamide est un PA12.

Selon un autre mode de mise en oeuvre préféré du procédé selon l'invention, le polymère ayant une ou plusieurs fonction(s) à hydrogène labile selon l'invention est un polyamide-imide, les fonctions à hydrogène labile sont alors des fonctions amides.

Le polyamide-imide vendu sous la dénomination Torlon® par la société Solvay est approprié.

### Diamètre moyen

Le polymère selon l'invention se présente sous forme de particule, pouvant être sous forme sphérique, pseudo-sphérique ou anguleuse. Généralement, la granulométrie de la particule est fonction du procédé de fabrication de celle-ci.

Le diamètre moyen de la particule mesuré selon la norme ISO 13319 peut aller de 1 à 200 µm, particulièrement est compris entre 1 et 200 µm, de préférence allant de 1 à 150µm, et plus particulièrement est compris entre 1 et 150 µm.

Avantageusement, Le diamètre moyen de la particule va de 1 à 100µm, de préférence de 5 à 100 µm, plus particulièrement de 5 à 60 µm, et encore plus particulièrement de 5 à 20 µm.

De préférence, le polymère sous forme de particule utilisé appartient à la gamme Orgasol® commercialisé par Arkema France.

### Agent de réticulation

L'agent de réticulation selon la présente invention comprend au moins deux fonctions capables de réagir avec une fonction à hydrogène labile du polymère.

En d'autres termes, l'agent de réticulation comporte au moins deux fonctions électrophiles, capables de réagir avec une fonction à hydrogène labile, c'est-à-dire nucléophile du polymère.

L'agent de réticulation polyfonctionnel selon l'invention permet ainsi de former des « ponts » entre les différentes chaînes macromoléculaires du polymère, qui se font nécessairement par des liaisons covalentes.

Les agents de réticulation comprenant au moins 2 fonctions susceptibles de réagir avec les fonctions du polymère sont de préférence des composés portant des fonctions isocyanate, carbodiimides, époxy, acyllactame, oxazoline et ses isomères, oxazine et ses isomères, PCl₃.

De préférence, les agents de réticulation possèdent des fonctions isocyanates, ce sont des polyisocyanates.

A titre d'agent de réticulation polyfonctionnel particulièrement préféré selon l'invention, on peut citer notamment lexylylène diisocynate, l'isophorone diisocyanate, le naphtalène-1,5-diisocyanate, le diphénylméthane-4,4'-diisocyanate, le triphénylméthane-4,4',4"-triisocyanate, le toluène-2,4-diisocyanate et les alkylène-diisocyanate, et plus préférentiellement l'hexaméthylène diisocyanate (noté HMDI).

A titre d'exemple, le schéma (I) suivante illustre une réaction de réticulation entre une chaîne polyamide et un agent de réticulation polyfonctionnel, ici l'hexaméthylène diisocyanate.

Une étape de catalyse a conduit à la formation d'un amidate, provenant d'une des fonctions à hydrogène labile du polymère.

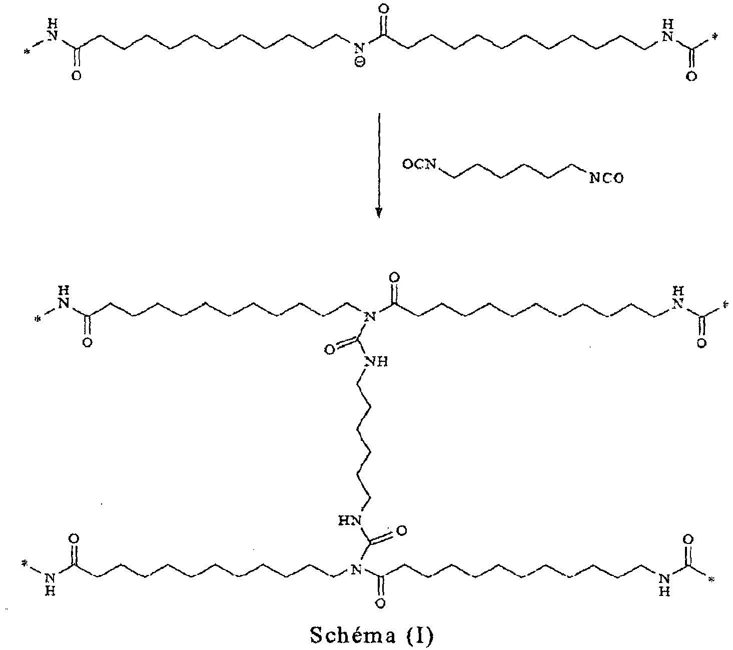

Selon un mode de mise en oeuvre du procédé selon l'invention, les polymères ayant une ou plusieurs fonction(s) à hydrogène labile selon l'invention peuvent être les polymères déjà réticulés, mais qui comportent encore des fonction(s) à hydrogène labile, aptes à réagir avec un agent de réticulation, identique ou différent de l'agent de réticulation déjà utilisé.

Le choix de l'agent de réticulation peut ainsi influencer les propriétés physico-chimiques du matériau final recherché.

L'agent de réticulation mis en oeuvre dans le procédé selon l'invention peut être en une teneur de 0,1 à 15% en moles, de préférence de 0,5 à 10%, moles par rapport au nombre de mole total des monomères constituant le polymère ayant une ou plusieurs fonction(s) à hydrogène labile selon l'invention.

### Etape d'ajout

L'étape d'ajout de l'agent de réticulation sur la poudre de polymère ayant une ou plusieurs fonction(s) à hydrogène labile selon l'invention peut se faire de manière suivante :
(1) soit en imprégnant l'agent de réticulation directement sur les particules, si celui-ci est liquide ou au sein d'un solvant, puis de chauffer afin de démarrer la réaction ;
(2) soit en remettant en dispersion solide/liquide la poudre de polymère dans un solvant de l'agent de réticulation puis d'y ajouter l'agent de réticulation à la température de réaction afin de démarrer la réaction de réticulation.

L'homme du métier est capable de choisir le solvant approprié pour l'agent de réticulation. On peut notamment citer des coupes d'hydrocarbures paraffiniques pour l'HMDI par exemple.

De préférence, le procédé selon l'invention ne met pas en oeuvre, ni ne conduit à un latex.

La réaction doit avoir lieu à une température inférieure à la fusion du polymère de sorte que la poudre ne s'agglomère pas.

### Etape de formation d'espèces réactives

Il se peut qu'une étape préalable de formation d'espèces réactives du polymère soit nécessaire. Afin de générer l'espèce réactive, c'est-à-dire nucléophile, il est possible d'utiliser une base suffisamment forte pour arracher les hydrogènes labiles du polymère.

Ces bases peuvent être choisies parmi l'hydrure de sodium, l'hydrure de potassium, le sodium, le méthylate et/ou l'éthylate de sodium.

La quantité de base peut en général varier de 0,1 à 3 moles pour 100 moles de polymère, et de préférence est comprise entre 0,1 et 3 moles pour 100 moles de polymère.

Ce procédé de réticulation en surface de particule de polymère conduit à des particules de polymère partiellement réticulé. En effet, la particule de polymère va comprendre une ou plusieurs zones de polymère réticulé, selon la teneur en agent de réticulation mis en oeuvre au cours du procédé.

Plus précisément, la zone de polymère réticulé est une couche plus ou moins épaisse, selon la teneur en agent de réticulation introduit et la durée de la réaction de réticulation, se situant sur la surface de la particule, c'est-à-dire sur un coeur en polymère ayant une ou plusieurs fonction(s) à hydrogène labile selon l'invention. La couche est alors un gradient de composition en polymère réticulé de plus en plus concentré en noeuds de réticulation en s'éloignant du coeur.

Selon un mode de mise en oeuvre du procédé selon l'invention, le polymère ayant une ou plusieurs fonction(s) à hydrogène labile est du polyamide et l'agent de réticulation est un polyisocyanate. Le procédé conduit alors à une poudre de structure coeur-écorce ayant un coeur en polyamide et une écorce en polyamide-imide de plus en plus concentrée en fonctions imide en s'éloignant du coeur.

Selon un autre mode de mise en oeuvre du procédé selon l'invention, le polymère ayant une ou plusieurs fonction(s) à hydrogène labile est du polyamide-imide et l'agent de réticulation est un polyisocyanate. Le procédé conduit alors à une poudre de structure coeur-écorce ayant un coeur en polyamide-imide et une écorce en polyamide-imide de plus en plus concentrée en fonctions imide en s'éloignant du coeur.

L'avantage que présente ce procédé est qu'il permet de contrôler l'épaisseur de la couche de polymère réticulé ainsi que la nature des « ponts » entre les chaines macromoléculaires grâce à la nature de l'agent de réticulation.

### Procédé de polymérisation

Le procédé de réticulation selon l'invention peut être mis en oeuvre sur des poudres de polymère, en tant que produit « fini ». Il peut également être incorporé à un procédé de polymérisation.

Lorsque le polymère selon l'invention est un polyamide, il peut par exemple constituer l'une des étapes finales du procédé de polycondensation ou bien d'un procédé de polymérisation anionique.

L'invention a également pour objet un procédé de polymérisation d'un polymère sous forme de poudre comprenant les étapes successives suivantes :
- une étape de polymérisation,
- une étape de réticulation telle que définie ci-dessus, du polymère sous forme de poudre obtenue à l'étape précédente, et
- une étape éventuelle de neutralisation du milieu réactionnel.

De préférence, l'étape de polymérisation est une étape de polymérisation anionique en solution dans un solvant, en présence d'un catalyseur et d'un activateur.

De préférence, la poudre est une poudre de polyamide. Plus particulièrement, le procédé vise la polymérisation d'amide, tel que la polymérisation de lactame 6, de lactame 12 ou de leur mélange. La polymérisation est effectuée en présence d'un catalyseur et d'un activateur.

S'agissant de la polymérisation anionique qui est mise en oeuvre pour l'obtention des particules de PA, celle-ci est conduite dans un solvant.

### Le Solvant

Le solvant utilisé dissout le monomère mais pas les particules de polymère qui se forment pendant la polymérisation. Des exemples de solvant sont donnés dans le brevet EP192515. Avantageusement, le solvant est une coupe d'hydrocarbures paraffiniques dont la plage d'ébullition va de 120 à 170°C et avantageusement est comprise entre 120 et 170°C, de préférence de 140 à 170°C, et avantageusement est comprise entre 140 et 170°C. Le solvant peut être sursaturé en monomère à la température d'initiation, c'est-à-dire à la température à laquelle débute la polymérisation.

Il est également possible de mettre en oeuvre la polymérisation dans un solvant non sursaturé en monomère. Dans ce cas, le milieu réactionnel contient le monomère dissous dans le solvant à une concentration éloignée de la sursaturation à la température d'initiation.

### Le Catalyseur

Un catalyseur est choisi parmi les catalyseurs usuels de la polymérisation anionique des lactames. Il s'agit d'une base suffisamment forte pour conduire dans le cas d'un lactame à un lactamate après réaction avec le lactame. Une combinaison de plusieurs catalyseurs est envisageable. A titre d'exemples non limitatifs, on peut citer l'hydrure de sodium, l'hydrure de potassium, le sodium, le méthylate et/ou l'éthylate de sodium. La quantité de catalyseur(s) introduite peut en général varier de 0,5 à 3 moles pour 100 moles de monomère, et avantageusement est comprise entre 0,5 et 3 moles pour 100 moles de monomère.

### L'Activateur

On ajoute également un activateur dont le rôle est de provoquer et/ou accélérer la polymérisation. L'activateur est choisi parmi les lactames-N-carboxyanilides, les (mono)isocyanates, les polyisocyanates, les carbodiimides, les cyanamides, les acyllactames et acylcarbamates, les triazines, les urées, les imides N-substituées, les esters et le trichlorure de phosphore. Il peut éventuellement aussi s'agir d'un mélange de plusieurs activateurs. L'activateur peut aussi éventuellement être formé *in situ*, par exemple, par réaction d'un isocyanate d'alkyle avec le lactame pour donner un acyllactame. Le rapport molaire catalyseur/activateur va de 0,2 à 2, avantageusement est compris entre 0,2 et 2, de préférence de 0,8 à 1,2, avantageusement est compris entre 0,8 et 1,2.

On peut ajouter dans le milieu réactionnel tout type de charges (pigments, colorants, noir de carbone, nanotubes de carbone...) ou additifs (antioxydants, anti-UV, plastifiants,...) à condition que tous ces composés soient bien secs et inertes vis-à-vis du milieu réactionnel.

### La polymérisation

La polymérisation anionique est conduite en continu ou bien de préférence, en discontinu (batch). En discontinu, on introduit le solvant, puis simultanément ou successivement le ou les monomère(s), le catalyseur et l'activateur. Il est recommandé d'introduire d'abord le solvant et le ou les monomère(s) puis d'éliminer toute trace d'eau, par exemple à l'aide d'une distillation azéotropique, puis d'ajouter le catalyseur une fois le milieu anhydre. Il peut être avantageux pour éviter la prise en masse ou la perte de contrôle de la polymérisation d'introduire l'activateur non pas en une seule fois mais par incréments ou bien à une vitesse d'introduction donnée. On opère à la pression atmosphérique ou bien sous une pression légèrement supérieure (pression partielle du solvant chaud) et à une température allant de 20°C à la température d'ébullition du solvant.

La température d'initiation et de polymérisation des lactames va en général de 70 à 150°C, avantageusement est compris entre 70 et 150°C, de préférence de 80 à 130°C avantageusement est compris entre 80 et 130, et avantageusement <120°C et >90°C.

Intervient ensuite la réaction de réticulation par ajout de l'agent de réticulation tel que défini ci-dessus.

La polymérisation se termine enfin par une étape de neutralisation.

### Particules de poudre

Les particules de poudre selon l'invention sont obtenues par le procédé de réticulation décrit ci-dessus Les particules selon l'invention sont également susceptibles d'être obtenue par le procédé réticulation décrit ci-dessus.

La particule de poudre de polymère selon l'invention comprend
- une ou plusieurs zones constituées de polymère possédant une ou plusieurs fonction(s) à hydrogène labile, tel que défini ci-dessus et
- une ou plusieurs zones comprenant ledit polymère réticulé par l'agent de réticulation tel que défini ci-dessus.

De préférence, la particule de poudre comprend
- un coeur constitué de polymère possédant une ou plusieurs fonction(s) à hydrogène labile, tel que défini ci-dessus et
- une couche, disposée sur ledit coeur, comprenant ledit polymère réticulé par l'agent de réticulation tel que défini ci-dessus.

La couche en polymère réticulé est un gradient de composition en polymère réticulé de plus en plus concentré en noeuds de réticulation en s'éloignant du coeur.

Selon un premier mode de réalisation, la couche en polymère réticulé peut être une couche externe, destinée à être en contact avec l'air. La particule selon ce mode de réalisation a alors une structure coeur-écorce.

Selon un second mode de réalisation, la couche en polymère réticulé peut constituer une couche intermédiaire, destinée à être intercalée au sein d'une structure multicouche. La couche réticulée peut se situer entre 2 couches de polymère éventuellement réticulé.

Selon un troisième mode de réalisation, la particule peut comporter plusieurs couches de polymère réticulé par des agents de réticulation différents.

La forme des particules selon l'invention est fonction de la forme des particules du polymère initial ayant une ou plusieurs fonction(s) à hydrogène labile.

L'épaisseur de la couche de polymère réticulé sera fonction de la quantité de l'agent de réticulation introduit au cours du procédé de réticulation et de la durée de l'étape de réticulation.

La granulométrie de la particule selon l'invention est fonction de la granulométrie de la particule de polymère initial ayant une ou plusieurs fonction(s) à hydrogène labile.

L'avantage du procédé de réticulation selon l'invention est qu'il conserve la dispersion granulométrique de la poudre qui est le réactif initial, c'est-à-dire la taille moyenne et la distribution resserrée. De plus, à la fusion, les particules de poudre selon l'invention conservent la forme de la particule initiale.

Cette propriété est très recherchée notamment dans le domaine des composites. Il est nécessaire d'avoir le plus de particules autour de la cible, et très peu, voire aucune très grosses particules, afin qu'elles puissent remplir leur rôle de spacer entre les feuillets de fibres de carbone dans les composites. De plus, la porosité de surface de la poudre initiale reste inchangée. Lorsque le polymère initial est cristallin, il a été observé que la cristallinité est également conservée.

Le polymère ayant une ou plusieurs fonction(s) à hydrogène labile et l'agent de réticulation sont tels que décrits ci-dessus pour le procédé de réticulation.

Selon un mode de réalisation particulier de l'invention, le polymère ayant une ou plusieurs fonction(s) à hydrogène labile est du polyamide et l'agent réticulant est un polyisocyanate, la particule de structure coeur-écorce aura un coeur en polyamide et une écorce en polyamide-imide de plus en plus concentré en fonctions imide en s'éloignant du coeur.

Selon un mode de réalisation préféré, le polymère ayant une ou plusieurs fonction(s) à hydrogène labile appartient à la gamme de produit Orgasol® et l'agent de réticulation est un polyisocyanate.

### Formulation

L'invention porte également sur une composition comprenant la poudre telle que définie ci-dessus. De préférence, la composition est thermodurcissable.

La poudre obtenue peut être utilisée telle qu'elle et est introduite dans une composition du type anhydre ou sous forme d'une émulsion huile dans eau (H/E) ou eau dans l'huile (E/H).

De préférence, la composition selon l'invention comprend une poudre telle que définie ci-dessus.

Lorsque la composition est utilisée dans le domaine cosmétique, elle comprend un milieu cosmétiquement acceptable, qui peut être de l'eau, un ou plusieurs alcools et leur mélange.

### Utilisation

L'invention concerne l'utilisation de la poudre telle que définie ci-dessus notamment en tant que charge ou renfort, dans des composites, dans des adhésifs structuraux, dans des revêtements de substrat, notamment à base de peinture poudre ou liquide qui peuvent être appliqués sur des substrats métalliques, plastiques, bois, verre, papier, caoutchouc, et dans des papiers transfert.

L'invention concerne également l'utilisation de la poudre telle que définie ci-dessus pour fabriquer des objets par agglomération de poudres par fusion provoquée par un rayonnement choisi parmi un faisceau laser, un rayonnement infra rouge ou un rayonnement UV.

L'invention concerne enfin l'utilisation de la poudre telle que définie ci-dessus en tant qu'additif dans des compositions cosmétiques.

Les exemples suivants servent à illustrer l'invention sans toutefois présenter un caractère limitatif.

### Exemples

Les caractéristiques physico-chimiques des poudres exemplifiées ont été évaluées selon les méthodes suivantes :

### Mesure du diamètre moyen :

La mesure du diamètre moyen des particules des poudres exemplifiées est réalisée selon la norme ISO 13319.

### Mesure des caractéristiques thermiques :

L'analyse des poudres est faite selon la norme ISO 11357-3 "Plastics : Differential scanning calorimetry (DSC) Part 3: Détermination of temperature and enthalpy of melting and crystallization."

### Mesure de l'enthalpie de fusion :

L'enthalpie de fusion est directement proportionnelle au taux de cristallinité du polymère. Une comparaison de l'enthalpie de fusion entre 2 produits permet donc de comparer leur taux de cristallinité.

L'enthalpie de fusion est mesurée par DSC selon la norme ISO 11357-3.

### Test de solubilité :

La solubilité est mesurée en introduisant 1g de poudre par litre d'hexafluoroisopropanol. Ces mélanges sont maintenus 24 heures à température ambiante afin de dissoudre les chaines de polymères non réticulées.

Ces solutions sont ensuite filtrées pour éliminer la partie insoluble, puis analysées par SEC à l'aide d'un appareil Waters Alliance 2695. Un détecteur Waters 2414 RID est utilisé. Le coefficient de réponse réfractométrique K(RI) est alors mesuré.

### 1. Synthèse d'une poudre polyamide 12 réticulée

On introduit dans le réacteur maintenu sous azote 2800 mL de solvant, puis successivement 899 g de lactame 12, 14,4 g d'EBS (Ethylène Bis Stéaramide) et 73,0 g d'Orgasol® 2002 UD NAT 1 (poudre de PA12). Après avoir mis en route l'agitation à 300t/min, on chauffe progressivement jusqu'à 110°C, puis on distille sous vide 280 mL de solvant afin d'entrainer par azéotropie toute trace d'eau qui pourrait être présente.

Après retour à la pression atmosphérique, on introduit alors rapidement sous azote le catalyseur anionique : 2,2 g d'hydrure de sodium à 60% de pureté dans de l'huile et on augmente l'agitation à 700 tr/min, sous azote à 105°C.

Ensuite, on reste à cette température pendant 30 minutes. Grâce à une pompe doseuse, on réalise une injection continue dans le milieu réactionnel de l'activateur choisi, à savoir l'isocyanate de stéaryle (38,3 g rempli à 153,0 g avec du solvant) selon le programme suivant : 51 g/h pendant 3 heures puis la température est élevée à 120°C pendant 2 heures.

L'étape de réticulation en surface de la poudre obtenue est réalisée comme suit. A 120°C sous agitation, une solution d'hexaméthylène diisocyanate : 5%mol d'HMDI par rapport au lactame 12 est ajoutée au milieu réactionnel pendant 3 heures, puis 2 heures supplémentaires sont laissées afin de terminer toutes les réactions.

La poudre est ensuite filtrée et séchée afin d'être neutralisée par une solution aqueuse de H₃PO₄. Puis la poudre neutralisée est à nouveau séchée.

Afin de vérifier l'efficacité de la réticulation, la poudre est plongée dans le m-crésol à 90°C, des particules insolubles apparaissent caractérisant la bonne réticulation du polyamide (pas d'insoluble dans le cas de l'Orgasol 2002 D Nat1).

### Evaluation de la poudre:

Deux types de poudre ont été comparés :
- une poudre commerciale de dénomination Orgasol® 2002 D NAT 1, non réticulée ; cette poudre constitue la poudre comparative A. Elle correspond à une poudre préparée suivant le procédé précédemment décrit mais sans l'étape de réticulation,
- la poudre selon l'invention notée B, qui est obtenue à partir du procédé selon l'exemple 1.

### Test de solubilité :

Le coefficient de réponse réfractométrique K(RI) est mesuré. Le K(RI) est directement proportionnel à la concentration de PA12 dans l'HFIP. Un coefficient de 201 est mesuré pour l'Orgasol® 2002 D Nat1, et un coefficient de 45 est mesuré pour la poudre de l'invention.

Les résultats figurent dans le tableau 1 ci-dessous.

**Tableau 1**

| | Poudre comparative (L12) | Poudre selon l'invention (L12) |
|---|---|---|
| | A | B |
| Diamètre moyen (µm) | 19,0 | 19,0 |
| Température de fusion (°C) | 180 | 183 |
| Enthalpie de fusion (J/g) | 113 | 108 |
| Solubilité dans l'HFIP (%) | 100 | 23 |

Ces résultats mettent en évidence le fait que la granulométrie de la poudre initiale est conservée.

Le test sur l'enthalpie de fusion montre que la réticulation de la poudre ne modifie en rien la cristallinité du matériau.

Les résultats relatifs à la solubilité des poudres montrent que la réticulation de la poudre a bien eu lieu. De plus, ce test met en évidence la résistance aux solvants de la poudre B selon l'invention.

### 2. Synthèse d'une poudre de PA6 réticulée

On introduit dans le réacteur maintenu sous azote 2452 mL de solvant, puis successivement 919,3 g de lactame 6 (caprolactame), 4,7 g d'EBS (Ethylène Bis Stéaramide) et 10,5 g d'Aerosyl® R972. Après avoir mis en route l'agitation à 300t/min, on chauffe progressivement jusqu'à 110°C, puis on distille sous vide 294 mL de solvant afin d'entrainer par azéotropie toute trace d'eau qui pourrait être présente.

Après retour à la pression atmosphérique, on introduit alors rapidement sous azote le catalyseur anionique : 6,3 g d'hydrure de sodium à 60% de pureté dans de l'huile et on augmente l'agitation à 900 tr/min, sous azote à 105°C. Ensuite, on reste à cette température pendant 30 minutes. Grâce à une pompe doseuse, on réalise une injection continue dans le milieu réactionnel de l'activateur choisi, à savoir l'isocyanate de stéaryle (27,3 g rempli à 147,1 g avec du solvant) selon le programme suivant : 49 g/h pendant 3 heures puis la réaction se déroule pendant encore 2 heures supplémentaires à 120°C.

L'étape de réticulation en surface de la poudre obtenue est réalisée comme suit. A 120°C sous agitation, une solution d'hexaméthylène diisocyanate : 5%mol d'HMDI par rapport au lactame 6 est ajoutée au milieu réactionnel pendant 3 heures, puis 2 heures supplémentaires sont laissées afin de consommer toutes les fonctions réactives.

La poudre est ensuite filtrée et séchée afin d'être neutralisée par une solution aqueuse de H₃PO₄. Puis la poudre neutralisée est à nouveau séchée.

### Evaluation de la poudre :

Deux types de poudre ont été comparés :
- une poudre commerciale de dénomination Orgasol® 1002 D Nat1, non réticulée ; cette poudre constitue la poudre comparative C. Elle correspond à une poudre préparée suivant le procédé précédemment décrit mais sans l'étape de réticulation,
- la poudre selon l'invention notée D, qui est obtenue à partir du procédé selon l'exemple 2.

### Test de solubilité

Les poudres sont plongées dans le m-crésol à 90°C. Des particules insolubles apparaissent caractérisant la bonne réticulation du polyamide D. Il n'y a pas apparition d'insoluble dans le cas de la poudre comparative C.

Le coefficient de réponse réfractométrique K(RI) est mesuré. Le K(RI) est directement proportionnel à la concentration de PA6 dans l'HFIP. Un coefficient de 209 est mesuré pour la poudre comparative C, et un coefficient de 82 est mesuré pour la poudre D selon l'invention.

Les résultats figurent dans le tableau 2 ci-dessous.

**Tableau 2**

| | Poudre comparative (PA6) C | Poudre selon l'invention (PA6) D |
|---|---|---|
| Diamètre moyen (µm) | 20,9 | 20,9 |
| Température de fusion (°C) | 210 | 205 |
| Enthalpie de fusion (J/g) | 116 | 109 |
| Solubilité dans l'HFIP (%) | 100 | 38 |

### 3. Synthèse d'une poudre de polyamide-imide réticulée

### Synthèse du polyamide :

On introduit dans le réacteur maintenu sous azote 2800 mL de solvant, puis successivement 919,3 g de lactame 12 (lauryllactame), 14,7 g d'EBS (Ethylène Bis Stéaramide) et 1,84 g de Sipernat® 320DS. Après avoir mis en route l'agitation à 300t/min, on chauffe progressivement jusqu'à 110°C, puis on distille sous vide 280 mL de solvant afin d'entrainer par azéotropie toute trace d'eau qui pourrait être présente.

Après retour à la pression atmosphérique, on introduit alors rapidement sous azote le catalyseur anionique : 2,36 g d'hydrure de sodium à 60% de pureté dans de l'huile et on augmente l'agitation à 800 tr/min, sous azote à 105°C. Ensuite, on reste à cette température pendant 30 minutes. Grâce à une pompe doseuse, on réalise une injection continue dans le milieu réactionnel de l'activateur choisi, à savoir l'isocyanate de stéaryle (27,3 g rempli à 147,1 g avec du solvant) selon le programme suivant : 49 g/h pendant 3 heures puis la température est élevée à 120°C pendant 2 heures.

### Synthèse du polyamide-imide :

L'obtention d'une poudre de polyamide-imide est réalisée comme suit. A 120°C sous agitation, une solution de methylenebis(cyclohexyl isocyanate) : 0,1%mol d'H12MDI par rapport au lactame 12 est ajoutée au milieu réactionnel pendant 3 heures, puis 2 heures supplémentaires sont laissées afin de consommer toutes les fonctions de l'agent réticulant. Nous obtenons alors une poudre de polyamide-imide complètement soluble dans le m-crésol à 90°C, qui constitue la poudre comparative E.

### Réticulation du polyamide-imide :

L'étape de réticulation en surface de la poudre obtenue est réalisée comme suit. A 120°C sous agitation, une solution d'hexaméthylène diisocyanate : 4,9%mol d'HMDI par rapport au lactame 12 est ajoutée au milieu réactionnel pendant 3 heures, puis 2 heures supplémentaires sont laissées afin de consommer toutes les fonctions réactives.

La poudre est ensuite filtrée et séchée afin d'être neutralisée par une solution aqueuse de H₃PO₄. Puis la poudre neutralisée est à nouveau séchée.

### Evaluation de la poudre :

Deux types de poudre ont été comparés :
- une poudre comparative E, issue de la synthèse du polyamide-imide. décrite ci-dessus ;
- la poudre selon l'invention notée F, qui est obtenue à partir du procédé selon l'exemple 3.

### Test de solubilité

Les poudres sont plongées dans le m-crésol à 90°C. Des particules insolubles apparaissent caractérisant la bonne réticulation du polyamide-imide F.

Les résultats figurent dans le tableau 3 ci-dessous.

**Tableau 3**

| | Poudre comparative (PAI) E | Poudre selon l'invention (PAI) F |
|---|---|---|
| Diamètre moyen (µm) | 49,4 | 49,4 |
| Température de fusion (°C) | 178 | 177 |
| Enthalpie de fusion (J/g) | 110 | 106 |
| Solubilité dans l'HFIP (%) | 100 | 30 |

Ces exemples montrent que, quelque soit le type de polymère utilisé la granulométrie de la poudre initiale et la cristallinité du matériau sont conservées.

## Revendications

1. Procédé de réticulation en surface d'un polymère, sous la forme de particule, possédant une ou plusieurs fonctions à hydrogène labile, ledit polymère étant choisi parmi les polymères comportant une ou plusieurs fonctions amide, les polyuréthanes, les polyamines et un mélange de ces polymères, comprenant une étape mettant en oeuvre un agent de réticulation comprenant au moins 2 fonctions susceptibles de réagir avec les fonctions à hydrogène labile du polymère, le procédé de réticulation se faisant à une température inférieure au point de fusion du polymère.

2. Procédé selon la revendication 1, **caractérisé en ce que** les polymères sont choisis parmi des polyamides, les polyamides-imides et leur mélange, de préférence, les polymères sont des polyamides.

3. Procédé selon la revendication 2, **caractérisé en ce que** les polyamides sont choisis parmi les PA6, PA11, PA12, PA6/12, PA6.12, PA6.10, PA10.10, PA10.12, PA6.6, PA8, PA4, PA4.6 ; et un mélange de ces polymères.

4. Procédé selon la revendication 3, **caractérisé en ce que** le polyamide est le PA12.

5. Procédé selon l'une quelconque des revendications précédentes, **caractérisé en ce que** les particules de polymère présentent un diamètre moyen allant de 1 à 200 µm, particulièrement est compris entre 1 et 200 µm, de préférence allant de 1 à 150µm, et plus particulièrement est compris entre 1 et 150 µm.

6. Procédé selon l'une quelconque des revendications précédentes, **caractérisé en ce que** l'agent de réticulation est choisi parmi des composés portant des fonctions isocyanate, carbodiimides, époxy, acyllactame, oxazoline et ses isomères, oxazine et ses isomères, PCl₃.

7. Procédé selon la revendication 6, **caractérisée en ce que** l'agent de réticulation est un polyisocyanate.

8. Procédé selon l'une quelconque des revendications précédentes, **caractérisé en ce que** l'agent de réticulation est en une teneur de 0,1 à 15% en moles, de préférence de 0,5 à 10%, moles par rapport au nombre de mole total des monomères constituant le polymère ayant une ou plusieurs fonction(s) à hydrogène labile.

9. Procédé de polymérisation de polymère comprenant les étapes successives suivantes :
- une étape de polymérisation,
- une étape de réticulation telle que définie à l'une quelconque des revendications précédentes, du polymère sous forme de poudre obtenue à l'étape précédente, et
- une étape éventuelle de neutralisation du milieu réactionnel.

10. Procédé selon la revendication 9, **caractérisé en ce que** l'étape de polymérisation est une étape de polymérisation anionique en solution dans un solvant, en présence d'un catalyseur et d'un activateur.

11. Particule de poudre de polymère comprenant :
- une ou plusieurs zones constituées de polymère possédant une ou plusieurs fonction(s) à hydrogène labile, tel que défini aux revendications 1 à 5 et
- une ou plusieurs zone(s) comprenant ledit polymère réticulé, par l'agent de réticulation tel que défini aux revendications 1, 6 à 8.

12. Particule selon la revendication 11, **caractérisé en ce qu'**elle comprend :
- un coeur constitué de polymère possédant une ou plusieurs fonction(s) à hydrogène labile, tel que défini aux revendications 1 à 5 et
- une couche comprenant ledit polymère réticulé, par l'agent de réticulation tel que défini aux revendications 1, 6 à 8.

13. Composition comprenant la particule de poudre définie en revendication 11 ou 12.

14. Utilisation de la particule de poudre telle que définie en revendication 11 ou 12, dans des composites, dans des adhésifs structuraux, dans des revêtements de substrat, dans des papiers transfert.

15. Utilisation de la particule de poudre telle que définie en revendication 11 ou 12 pour fabriquer des objets par agglomération de poudres par fusion provoquée par un rayonnement choisi parmi un faisceau laser, un rayonnement infra rouge ou un rayonnement UV.

16. Utilisation de la particule de poudre telle que définie en revendication 11 ou 12, en tant qu'additif dans des compositions cosmétiques.

## Patentansprüche

1. Verfahren zur Oberflächenvernetzung eines Polymers in Teilchenform, das eine oder mehrere labilen Wasserstoff enthaltenden Funktionen aufweist, wobei das Polymer aus Polymeren mit einer oder mehreren Amidfunktionen, Polyurethanen, Polyaminen und einer Mischung dieser Polymere ausgewählt wird, umfassend einen Schritt der Verwendung eines Vernetzungsmittels mit mindestens 2 Funktionen, die mit den labilen Wasserstoff enthaltenden Funktionen des Polymers reagieren können, wobei das Vernetzungsverfahren bei einer Temperatur unterhalb des Schmelzpunkts des Polymers erfolgt.

2. Verfahren nach Anspruch 1, **dadurch gekennzeichnet, dass** die Polymere aus Polyamiden, Polyamidimiden und Mischungen davon ausgewählt werden und es sich bei den Polymeren vorzugsweise um Polyamide handelt.

3. Verfahren nach Anspruch 2, **dadurch gekennzeichnet, dass** die Polyamide aus PA6, PA11, PA12, PA6/12, PA6.12, PA6.10, PA10.10, PA10.12, PA6.6, PA8, PA4 und PA4.6 und einer Mischung dieser Polymere ausgewählt werden.

4. Verfahren nach Anspruch 3, **dadurch gekennzeichnet, dass** es sich bei dem Polyamid um PA12 handelt.

5. Verfahren nach einem der vorhergehenden Ansprüche, **dadurch gekennzeichnet, dass** die Polymerteilchen einen mittleren Durchmesser im Bereich von 1 bis 200 µm, insbesondere zwischen 1 und 200 µm, vorzugsweise im Bereich von 1 bis 150 µm und spezieller zwischen 1 und 150 µm liegt.

6. Verfahren nach einem der vorhergehenden Ansprüche, **dadurch gekennzeichnet, dass** das Vernetzungsmittel aus Verbindungen ausgewählt wird, die die folgenden Funktionen tragen: Isocyanat, Carbodiimid, Epoxid, Acyllactam, Oxazolin und Isomere davon, Oxazin und Isomere davon, PCl₃.

7. Verfahren nach Anspruch 6, **dadurch gekennzeichnet, dass** es sich bei dem Vernetzungsmittel um ein Polyisocyanat handelt.

8. Verfahren nach einem der vorhergehenden Ansprüche, **dadurch gekennzeichnet, dass** das Vernetzungsmittel in einem Gehalt von 0,1 bis 15 Mol-%, vorzugsweise von 0,5 bis 10 Mol-%, bezogen auf die Gesamtmolzahl der Monomere, aus denen das Polymer mit einer oder mehreren labilen Wasserstoff enthaltenden Funktionen besteht, vorliegt.

9. Verfahren zur Polymerisation eines Polymers, das die folgenden aufeinanderfolgenden Schritte umfasst:
- einen Schritt der Polymerisation,
- einen Schritt der Vernetzung des im vorhergehenden Schritt erhaltenen Polymers in Pulverform gemäß einem der vorhergehenden Ansprüche und
- einen fakultativen Schritt der Neutralisation des Reaktionsmediums.

10. Verfahren nach Anspruch 9, **dadurch gekennzeichnet, dass** es sich bei dem Schritt der Polymerisation um einen Schritt der anionischen Polymerisation in Lösung in einem Lösungsmittel in Gegenwart eines Katalysators und eines Aktivators handelt.

11. Polymerpulverteilchen, umfassend:
- einen oder mehrere Bereiche, die aus Polymer mit einer oder mehreren labilen Wasserstoff enthaltenden Funktionen gemäß den Ansprüchen 1 bis 5 bestehen, und
- einen oder mehrere Bereiche, die das mit dem Vernetzungsmittel vernetzte Polymer gemäß den Ansprüchen 1 und 6 bis 8 umfassen.

12. Teilchen nach Anspruch 11, **dadurch gekennzeichnet, dass** es Folgendes umfasst:
- einen Kern, der aus Polymer mit einer oder mehreren labilen Wasserstoff enthaltenden Funktionen gemäß den Ansprüchen 1 bis 5 besteht, und
- eine Schicht, die das mit dem Vernetzungsmittel vernetzte Polymer gemäß den Ansprüchen 1 und 6 bis 8 umfasst.

13. Zusammensetzung, umfassend das Pulverteilchen gemäß Anspruch 11 oder 12.

14. Verwendung des Pulverteilchens gemäß Anspruch 11 oder 12 im Verbundwerkstoffen, in Strukturklebstoffen, in Substratbeschichtungen und in Transferpapieren.

15. Verwendung des Pulverteilchen gemäß Anspruch 11 oder 12 zur Herstellung von Objekten durch Agglomeration von Pulvern durch Schmelzen, welches durch Strahlung, die aus einem Laserstrahl, Infrarotstrahlung oder UV-Strahlung ausgewählt wird, herbeigeführt wird.

16. Verwendung des Pulverteilchens gemäß Anspruch 11 oder 12 als Additiv in kosmetischen Zusammensetzungen.

## Claims

1. Process for the surface crosslinking of a polymer, in particle form, having one or more labile-hydrogen functions, said polymer being chosen from polymers comprising one or more amide functions, polyurethanes, polyamines and a blend of these polymers, comprising a step using a crosslinking agent comprising at least two functions capable of reacting with the labile-hydrogen functions of the polymer, the crosslinking process being carried out at a temperature lower than the melting point of the polymer.

2. Process according to Claim 1, **characterized in that** the polymers are chosen from polyamides and polyamide-imides and a blend thereof; preferably, the polymers are polyamides.

3. Process according to Claim 2, **characterized in that** the polyamides are chosen from PA6, PA11, PA12, PA6/12, PA6.12, PA6.10, PA10.10, PA10.12, PA6.6, PA8, PA4 and PA4.6, and a blend of these polymers.

4. Process according to Claim 3, **characterized in that** the polyamide is PA12.

5. Process according to any one of the preceding claims, **characterized in that** the polymer particles have an average diameter which ranges from 1 to 200 µm, which is particularly between 1 and 200 µm, which preferably ranges from 1 to 150 µm, and which more particularly is between 1 and 150 µm.

6. Process according to any one of the preceding claims, **characterized in that** the crosslinking agent is chosen from compounds bearing the following functions: isocyanate, carbodiimide, epoxy, acyllactam, oxazoline and its isomers, oxazine and its isomers, and PCl₃.

7. Process according to Claim 6, **characterized in that** the crosslinking agent is a polyisocyanate.

8. Process according to any one of the preceding claims, **characterized in that** the crosslinking agent is in a content of from 0.1 to 15 mol%, preferably from 0.5 to 10 mol%, relative to the total number of moles of the monomers constituting the polymer having one or more labile-hydrogen function(s).

9. Process for the polymerization of a polymer, comprising the following successive steps:
- a polymerization step,
- a step of crosslinking, as defined in any one of the preceding claims, of the polymer in powder form obtained in the previous step, and
- an optional step of neutralization of the reaction medium.

10. Process according to Claim 9, **characterized in that** the polymerization step is a step of anionic polymerization in solution in a solvent, in the presence of a catalyst and of an activator.

11. Particle of polymer powder comprising:
- one or more areas consisting of polymer having one or more labile-hydrogen function(s), as defined in Claims 1 to 5, and
- one or more area(s) comprising said polymer crosslinked with the crosslinking agent as defined in Claims 1 and 6 to 8.

12. Particle according to Claim 11, **characterized in that** it comprises:
- a core consisting of polymer having one or more labile-hydrogen function(s), as defined in Claims 1 to 5, and
- a layer comprising said polymer crosslinked with the crosslinking agent as defined in Claims 1 and 6 to 8.

13. Composition comprising the particle of powder defined in Claim 11 or 12.

14. Use of the particle of powder as defined in Claim 11 or 12, in composites, in structural adhesives, in substrate coatings and in transfer papers.

15. Use of the particle of powder as defined in Claim 11 or 12, for producing objects by agglomeration of powders by fusion brought about by radiation chosen from a laser beam, infrared radiation or UV radiation.

16. Use of the particle of powder as defined in Claim 11 or 12, as an additive in cosmetic compositions.
